# EUROPEAN PATENT APPLICATION

(11) **EP 4 830 740 A1**
(43) Date of publication of application: **28.10.2026**
(21) Application number: 25204631.3
(22) Date of filing: 25.09.2025
(51) Int. Cl.: A61L 27/24, A61L 27/52, A61L 31/04, A61L 31/14

(54) **RECOMBINANT COLLAGEN GEL MATERIAL, PREPARATION METHOD THEREFOR AND USE THEREOF**

(30) Priority: 21.04.2025 CN 202510503495
(71) Applicant: WitKang Zhiyuan Medical Devices (Xi'an) Co., Ltd., Xi'an City Shaanxi 710075 (CN)
(72) Inventor: LI, Yanting, Xi'an City, Shaanxi Province, 710075 (CN); WANG, Meihua, Xi'an City, Shaanxi Province, 710075 (CN); BAI, Xueni, Xi'an City, Shaanxi Province, 710075 (CN); HOU, Jianing, Xi'an City, Shaanxi Province, Xi'an City, Sha (CN); CHEN, Liang, Xi'an City, Shaanxi Province, 710075 (CN); WANG, Ya, Xi'an City, Shaanxi Province, 710075 (CN); LI, Yuan, Xi'an City, Shaanxi Province, 710075 (CN)
(74) Representative: IP TRUST SERVICES

(57) **Abstract**

The present disclosure provides a recombinant collagen gel material, a preparation method therefor, and the use thereof. The preparation method for the material comprises: mixing a recombinant collagen with a first aqueous phase to obtain a recombinant collagen solution and drying and granulating the recombinant collagen solution to obtain a first recombinant collagen microsphere; physically cross-linking the first recombinant collagen microsphere to obtain a second recombinant collagen microsphere; mixing the second recombinant collagen microsphere with a second aqueous phase under stirring and swelling the mixture to obtain a white dilute solution with suspended particles; and subjecting the white dilute solution with suspended particles to low-temperature irradiation cross-linking to obtain the recombinant collagen gel material. The preparation method of the present disclosure has shortened production cycle and greatly reduced production cost, and a gel material prepared by the method exhibits a high biosafety and good mechanical properties and degradation performance.

## Description

### TECHNICAL FIELD

The present disclosure belongs to the field of biomedical materials, relates to the field of injections for medical aesthetic filling, and specifically relates to a recombinant collagen gel material, a preparation method thereof, and the use thereof.

### BACKGROUND

Hydrogel is a three-dimensional network biomaterial composed of hydrophilic polymer chains, which can swell but does not dissolve in water, has the advantages such as strong water absorption, water retention, biocompatibility, etc., and has been widely studied in the fields such as biology, medical engineering, etc. As a biomedical material, it has been used in biopharmaceutical fields such as implantable medical devices, medical wound dressings, and drug release carriers. Hydrogel, among them, as a soft tissue filling material, can repair cavities and defective tissues caused by soft tissue injuries or lesions, remove wrinkles or improve facial contours in plastic surgery and medical aesthetic industry, etc.

A recombinant collagen is a collagen produced by genetic engineering technology. Compared with natural extracted collagen, it has a higher purity, a better biosafety, and a more controllable production process. Preparation methods for recombinant collagen hydrogels mainly include physical cross-linking, chemical cross-linking, enzymatic cross-linking, etc. The physical cross-linking method achieves physical cross-linking by repeated freezing and thawing cycles or noncovalent bonds (such as hydrogen bonding and hydrophobic interaction). The recombinant collagen hydrogel thus prepared has better mechanical properties but suffer from shortcomings such as a complicated process and difficult control of cross-linking degree. Hydrogels prepared by the chemical cross-linking method usually have residual cross-linking agents, initiators, etc., which may have a risk of inducing inflammation or cytotoxicity. The enzymatic cross-linking method is a method to realize cross-linking between polymers or protein molecules by using specific enzyme-catalyzed reactions. Although it is a green and efficient cross-linking method, it has some difficulties in scale production due to the relatively high production cost.

In summary, existing preparation methods for recombinant collagen hydrogels have problems, such as a complicated process, difficult control of cross-linking degree, residual chemical cross-linking agents, and a relatively high cost.

### SUMMARY

In order to solve the above technical problems, the present disclosure provides a recombinant collagen gel material, a preparation method therefor, and the use thereof. The preparation method of the present disclosure can greatly reduce the production cost and shorten the production cycle, has a high production efficiency and a strong operability, and is easy for scale production. The obtained material exhibits good mechanical properties, safety, non-toxicity, biodegradability, excellent biocompatibility, and no viral hazard.

In order to achieve the above objective, in one aspect, the present disclosure provides a method for preparing a recombinant collagen gel material, comprising:
mixing a recombinant collagen with a first aqueous phase to obtain a recombinant collagen solution and drying and granulating the recombinant collagen solution to obtain a first recombinant collagen microsphere;
physically cross-linking the first recombinant collagen microsphere to obtain a second recombinant collagen microsphere;
mixing the second recombinant collagen microsphere with a second aqueous phase under stirring and swelling the mixture to obtain a white dilute solution with suspended particles; and
subjecting the white dilute solution with suspended particles to low-temperature irradiation cross-linking to obtain the recombinant collagen gel material.

According to a specific embodiment of the present disclosure, the recombinant collagen gel material is a colorless and transparent jelly-like gel.

The recombinant collagen described in the present disclosure is a recombinant human-derived collagen disclosed in CN 108070032 B [METHOD FOR PURIFYING RECOMBINANT HUMAN-DERIVED COLLAGEN].

According to a specific embodiment of the present disclosure, the amino acid sequence of the recombinant collagen is as set forth in SEQ ID No: 1.

The recombinant collagen raw materials described in the specific embodiments of the present disclosure are all proteins with the amino acid sequence as set forth in SEQ ID No: 1 in the sequence listing.

According to a specific embodiment of the present disclosure, in the recombinant collagen solution, the mass fraction of the recombinant collagen is 10%-20%.

According to a specific embodiment of the present disclosure, the pH of the recombinant collagen solution is less than 7, more preferably, the pH is 4-6. According to a specific embodiment of the present disclosure, in the white dilute solution with suspended particles, the mass fraction of the second recombinant collagen microsphere is 2%-4%.

In the preparation method of the present disclosure, the content of the second recombinant collagen microsphere in the white dilute solution with suspended particles is just the content of the recombinant collagen in the recombinant collagen gel material in the present disclosure.

According to a specific embodiment of the present disclosure, the drying and granulating are carried out using a spray dryer with a nozzle diameter of 0.75-1.5 mm at a spray temperature of 140-260°C at a feed rate of 200-500 ml/h.

According to a specific embodiment of the present disclosure, the physical cross-linking is performed at a temperature of 120-160°C for a time period of 2-5 hours; preferably, the physical cross-linking is performed at a temperature of 120-140°C or 140-160°C for a time period of 2-3 hours or 3-5 hours.

According to a specific embodiment of the present disclosure, the physical cross-linking is performed at a vacuum degree of 0.08 MPa- (-0.1 MPa).

According to a specific embodiment of the present disclosure, the mixing under stirring is performed at a rotating speed of 80-120 r/min for a time period of 30-60 min.

According to a specific embodiment of the present disclosure, the swelling is performed at a temperature of 2-8°C for a time period of 6-10 hours.

According to a specific embodiment of the present disclosure, for the irradiation cross-linking, an electron beam or ⁶⁰Co-γ ray is used, and the irradiation dose is 10-25 kGy.

According to a specific embodiment of the present disclosure, when the white dilute solution with suspended particles is subjected to irradiation cross-linking, the white dilute solution with suspended particles has a temperature of 2-8°C, preferably 2-4°C.

According to a specific embodiment of the present disclosure, the first aqueous phase or second aqueous phase is selected from one of or a combination of two or more of ultrapure water, water for injection, and a phosphate buffer.

In another aspect, the present disclosure further provides a recombinant collagen gel material prepared by the above preparation method.

The present disclosure provides a recombinant collagen gel material, wherein based on the total mass of the recombinant collagen gel material being 100%, the content of the recombinant collagen is 2%-4%, and the content of the aqueous phase is 96%-98%;
the amino acid sequence of the recombinant collagen is as set forth in SEQ ID No: 1; and
the recombinant collagen gel material is a colorless and transparent jelly-like gel with a porous net-like structure inside.

After the recombinant collagen gel material of the present disclosure is freeze-dried, the cross section of the gel material has a porous net-like structure having honeycomb-shaped and dense pores, which is beneficial to the diffusion of nutrients and the promotion of cell proliferation.

According to a specific embodiment of the present disclosure, the aqueous phase is selected from one of or a combination of two or more of ultrapure water, water for injection, and a phosphate buffer.

According to a specific embodiment of the present disclosure, the pushing force for the recombinant collagen gel material to pass through a 27G needle is 15-20 N, more preferably 16-18 N.

In the present disclosure, the conditions for measuring the pushing force are filling with 2 mL of a recombinant collagen gel material sample, fitting a 27G needle, pushing at a constant speed of 10 mm/min until the sample is completely pushed out with a constant force value, thus completing the experiment. During the experiment process, the maximum force value is determined as the pushing force.

According to a specific embodiment of the present disclosure, the osmotic pressure of the recombinant collagen gel material is 270-310 mOsm/L, which is close to the ideal state of human physiology and activity.

According to a specific embodiment of the present disclosure, the recombinant collagen gel material has a rotational viscosity of 6000-9000 cp.

In the present disclosure, the conditions for measuring rotational viscosity are taking 200 ml of a test sample, using a No.5 rotor and a rotating speed of 25 rpm, and performing viscosity detection by a rotational viscometer at 25°C.

In another aspect, the present disclosure further provides the above preparation method, or a recombinant collagen gel material prepared by the above preparation method, or the above recombinant collagen gel material for use in the preparation of a medical material.

According to a specific embodiment of the present disclosure, preferably, the medical material is used for medical aesthetic water light therapy, medical aesthetic filling, soft tissue repair, or adhesion prevention.

Compared with the prior art, the present disclosure has the following beneficial effects.

Firstly, in the present disclosure, two-stage cross-linking is used, wherein the primary physical cross-linking is semi-cross-linking. By means of the semi-cross-linking, the denaturation temperature of the collagen, i.e., the temperature at which the helical structure unwinds, can be increased. After the collagen is dehydrated, cross-linking points are only formed between some collagen molecular chains. The secondary cross-linking is carried out by low-temperature irradiation cross-linking, so that the amino acid residues in the recombinant collagen generate free radicals and recombine in a very short time. The low temperature can slow down the diffusion rate of free radicals and promote the formation of a cross-linked network, thereby realizing the secondary cross-linking, thus preparing the recombinant collagen gel material. There is no need to introduce an additional chemical cross-linking agent during the production process, and the present disclosure exhibits a high biosafety, safety, non-toxicity, and excellent biocompatibility.

Secondly, the low-concentration recombinant collagen microspheres can achieve the effect of preparing a material with a high-concentration raw material, which greatly reduces the production cost, provides a strong operability, and is easy for scale production.

Thirdly, spray drying shortens the production cycle and improves the production efficiency compared with freeze drying.

Fourthly, the use of low-temperature irradiation cross-linking can achieve the effects of preventing bacterial contamination, prolonging storage time, and ensuring the good quality of the hydrogel.

Fifthly, the use of two-stage cross-linking can greatly enhance the mechanical properties and biodegradability of the material.

Sixthly, the present disclosure has a single component, only contains one recombinant collagen component, and has a high biosafety.

### BRIEF DESCRIPTION OF THE DRAWINGS

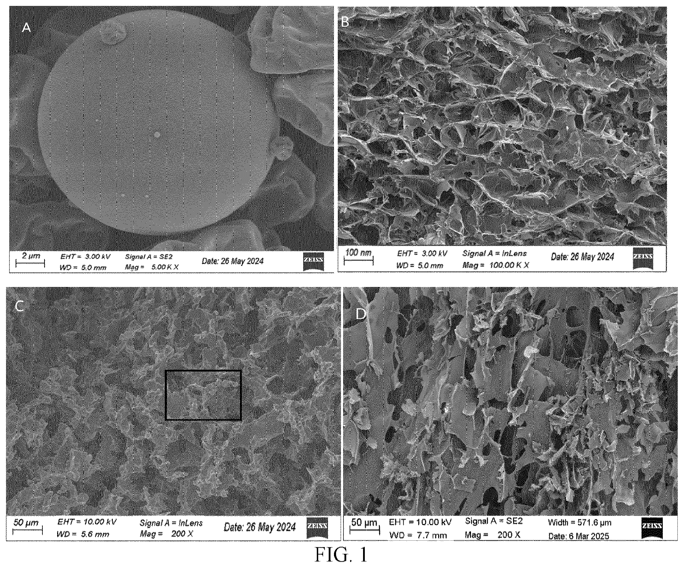

FIG. 1 shows electron microscope images of a material prepared in Example 1, wherein panel A is a scanning electron microscope image (5000×) of first recombinant collagen microspheres; panel B is a scanning electron microscope image (100000×) of the internal porous structure of the first recombinant collagen microspheres; panel C is a scanning electron microscope image (200×) of cross sections of the freeze-dried recombinant collagen gel material prepared in Example 1 after primary cross-linking; panel D is a scanning electron microscope image (200×) of cross sections of the freeze-dried recombinant collagen gel material prepared in Example 1 after secondary cross-linking in another batch of experiments.
FIG. 2 shows comparison images of the appearances of the samples prepared after primary cross-linking and secondary cross-linking of the recombinant collagen gel material prepared in Example 1;
FIG. 3 shows the morphologies of prepared recombinant collagen gel material samples observed after the samples are filled into syringes and then injected into glass dishes, wherein panel A is the morphology of the sample of Example 1 after injection, panel B is the morphology of the sample of Comparative Example 4 after injection, and panel C is the morphology of the sample of Comparative Example 7 after injection;
FIG. 4 is a test of the recombinant collagen gel material prepared in Example 1;
FIG. 5 shows images of the therapeutic effect of the recombinant collagen gel material prepared in Example 1 on a photoaged model;
FIG. 6 shows images of the therapeutic effect of a recombinant collagen gel material prepared in Comparative Example 1 on a photoaged model;
FIG. 7 shows the statistical results of skin elasticity, collagen content, and moisture of a photoaged murine model treated with the recombinant collagen gel material prepared in Example 1 at 0w, 1w, and 2w;
FIG. 8 shows the statistical results of skin elasticity, collagen content, and moisture of a photoaged murine model treated with the recombinant collagen gel material prepared in Comparative Example 1 at 0w, 1w, and 2w; and
FIG. 9 shows hematoxylin-eosin staining pictures of an aged murine model treated with the recombinant collagen gel material prepared in Example 1 at 2w.

### DETAILED DESCRIPTION

In order to understand the technical features, objectives and beneficial effects of the present disclosure more clearly, the technical solutions of the present disclosure are described in detail as below, but cannot be construed as limitations on the implementable scope of the present disclosure.

During actual production, those skilled in the art can all understand that some related process steps can be adjusted or increased or decreased on the premise that a qualified product can be obtained.

In order to make the objectives, technical solutions, and advantages of the embodiments of the present disclosure clearer, the technical solutions in the embodiments of the present disclosure will be described below clearly and completely with reference to the accompanying drawings in the embodiments of the present disclosure. Apparently, the described embodiments are only some, rather than all, of the embodiments of the present disclosure. Based on the embodiments of the present disclosure, all other embodiments that can be obtained by those of ordinary skill in the art without involving creative efforts shall fall within the scope of protection of the present disclosure.

The amino acid sequence of the recombinant collagen used in the following examples is:

GPPGEPGNPGKPGSPGPAGSNGEPGPAGSPGEKGSQGSNGNPGPAGNQGQPGNKGSPG
NPGKPGEPGSNGPQGEPGSQGNPGKNGQPGSPGSQGSPGNQGQPGKPGQPGEQGSPGNQG
PAGNEGPKGQPGQNGKPGSPGPPGEPGNPGKPGSPGPAGSNGEPGPAGSPGEKGSQGSNGN
PGPAGNQGQPGNKGSPGNPGKPGEPGSNGPQGEPGSQGNPGKNGQPGSPGSQGSPGNQGQ
PGKPGQPGEQGSPGNQGPAGNEGPKGQPGQNGKPGTPGPPGEPGNPGKPGSPGPAGSNGEP
GPAGSPGEKGSQGSNGNPGPAGNQGQPGNKGSPGNPGKPGEPGSNGPQGEPGSQGNPGKN
GQPGSPGSQGSPGNQGQPGKPGQPGEQGSPGNQGPAGNEGPKGQPGQNGKP (SEQ ID No:
1).

### Example 1

This example provided a method for preparing a recombinant collagen gel material, wherein:
(1) 100 g of a recombinant human collagen (a protein with an amino acid sequence as set forth in SEQ ID No: 1) was added into 900 g of water for injection and stirred by a magnetic stirrer for 2 hours to obtain a 10% recombinant collagen solution, wherein the pH of the recombinant collagen solution was 5.15.
(2) The above recombinant collagen solution with pH < 7 was subjected to spray granulation by a spray drier to obtain 80 g of first recombinant collagen microspheres, wherein the spray temperature was 150°C, the feed rate was 300 ml/h, the nozzle diameter was 1 mm, and the spray drying time was 2 hours.
(3) 2 g of the first recombinant collagen microspheres were taken and physically cross-linked for primary semi-cross-linking to obtain 2 g of second recombinant collagen microspheres, wherein the cross-linking conditions were: temperature: 140°C in a vacuum drying oven, time: 3 hours, and vacuum degree: -0.1 MPa.
(4) 2 g of the second recombinant collagen microspheres were added into 98 g of water for injection, mixed under stirring for 30 minutes at a rotating speed of 100 r/min, and then swelled in a refrigerator at 4°C for 6 hours to obtain a white dilute solution with suspended particles.
(5) The white dilute solution with suspended particles was filled in a vial, which was placed in a foam box containing an ice pack (used for maintaining 4°C) for irradiation sterilization, i.e., secondary low-temperature irradiation cross-linking to thus obtain the colorless and transparent jelly-like recombinant collagen gel material of Example 1 of the present disclosure, wherein a high energy electron beam was used for the irradiation cross-linking, and the irradiation dose was 10 kGy.

### Examples 2-5

Examples 2-5 provided a method for preparing a recombinant collagen gel material.

The conditions thereof were the same as in Example 1, except that the temperature for the physical cross-linking of the first recombinant collagen microspheres, and the concentration, swelling conditions, and irradiation cross-linking conditions of the second recombinant collagen microspheres are as shown in Table 1. The colorless and transparent jelly-like recombinant collagen gel materials of Examples 2-5 were thus prepared.

### Comparative Examples 1-5

Comparative Examples 1-5 provided a method for preparing a recombinant collagen gel material.

The conditions thereof were the same as in Example 1, except that the temperature for the physical cross-linking of the first recombinant collagen microspheres, and the concentration, swelling conditions, and irradiation cross-linking conditions of the second recombinant collagen microspheres are as shown in Table 1. The recombinant collagen gel materials of Comparative Examples 1-5 were thus prepared. The recombinant collagen in Comparative Example 1 was a commercially available recombinant type III humanized collagen (purchased from Xi'an DeNovo Hith Medical Technology Co., Ltd., batch No.: 2308B01, model: RHC-III-Lel).

### Comparative Example 6

Comparative Example 6 provided a method for preparing a recombinant collagen gel material.

The conditions thereof were the same as in Example 1, except that 1. the recombinant collagen solution was freeze-dried to form a recombinant collagen sponge, which was then physically cross-linked, frozen in liquid nitrogen for 3-5 min, and then ground in a mortar to obtain a recombinant collagen powder, and the recombinant collagen powder was mixed with water for injection to obtain white dilute solution with suspended particles.

### Comparative Example 7

Comparative Example 7 provided a method for preparing a recombinant collagen gel material.

The conditions thereof were the same as in Example 1, except that the material prepared in Comparative Example 7 was prepared according to a current conventional method (high concentration), wherein the temperature for the physical cross-linking was high, the concentration of the recombinant collagen microspheres was relatively high, the swelling time was relatively long, and the prepared material was subjected to terminal sterilization by irradiation sterilization or moist heat sterilization. No secondary cross-linking occurred during this process. The temperature for the physical cross-linking of the first recombinant collagen microspheres and the concentration, swelling conditions, and irradiation cross-linking conditions of the second recombinant collagen microspheres are as shown in Table 1. The recombinant collagen gel material of Comparative Example 7 was thus prepared.

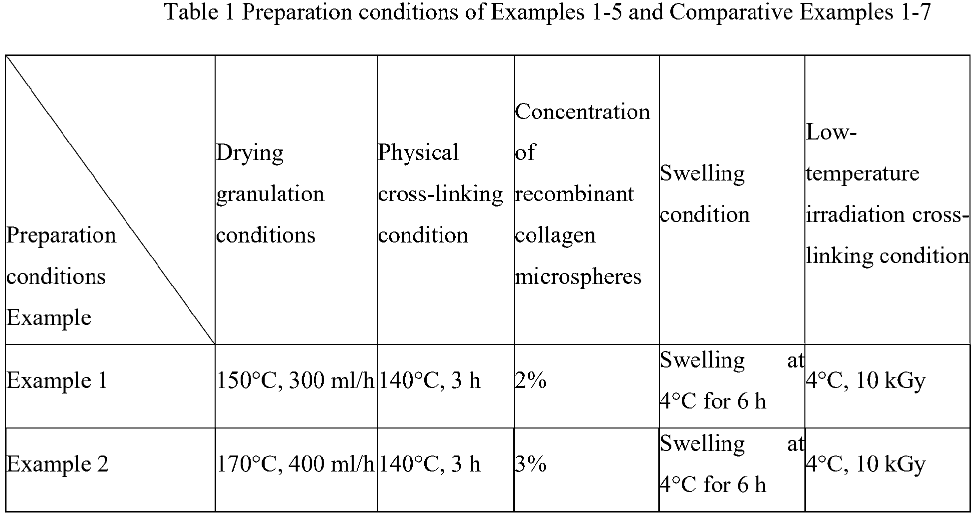

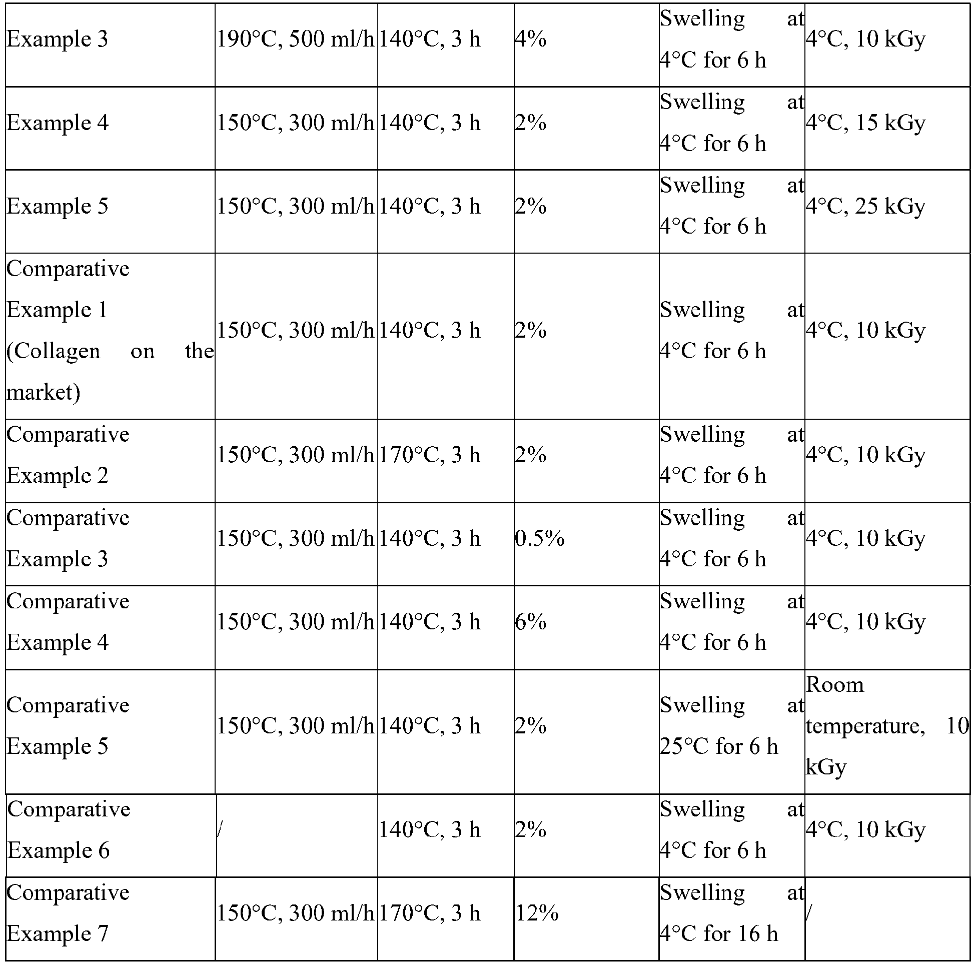

### Effect Example

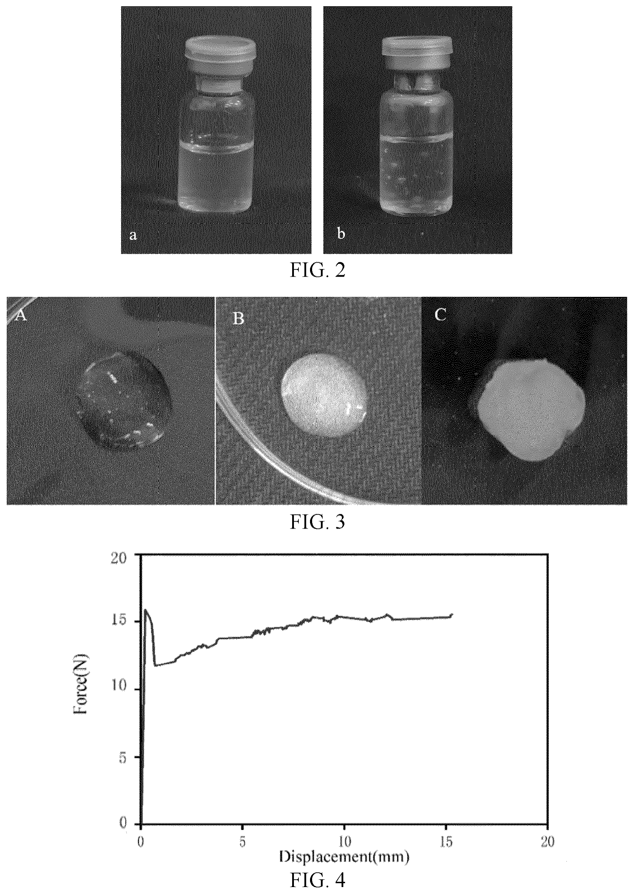

The following performance tests were carried out on the recombinant collagen gel materials prepared in the above examples and comparative examples:
1. Scanning electron microscope: The spray-granulated recombinant collagen microspheres, the prepared white dilute solution with suspended particles, and the recombinant collagen gel material in Example 1 were freeze-dried, put into liquid nitrogen for 3-5 min, and then separated apart, and the cross section was subjected to microscopic morphology observation by a scanning electron microscope. The results are as shown in FIG. 1: A: after spray granulation, the recombinant collagen had a microsphere structure with a diameter of about 10 µm; B shows a porous network structure inside the microsphere; C: cross-linking points were formed between some collagen molecular chains in the white dilute solution with suspended particles (as shown in the box); and D: the cross section of the recombinant collagen gel material had a porous net-like structure having honeycomb-shaped and dense pores, which was beneficial to the diffusion of nutrients and the promotion of cell proliferation.
2. Appearance test method: The appearances of products prepared before and after secondary cross-linking in Examples 1-5 and Comparative Examples 1-7 were compared. The comparison results are as shown in Table 2. The appearance comparison of the products prepared before and after secondary cross-linking in Example 1 is shown in FIG. 2, in which panel a in FIG. 2 shows the state of the prepared solution after primary physical semi-cross-linking and swelling, and panel b in FIG. 2 shows the state after low-temperature irradiation cross-linking.
3. Morphology of sample after injection: The gel materials prepared in Examples 1-5 and Comparative Examples 4 and 6 were filled into syringes and injected into glass dishes, and the morphologies of the samples were observed. The results are as shown in Table 2. The morphology of the sample of Example 1 after injection is as shown in panel A of FIG. 3, and the morphologies of the samples of the rest Examples 2-5 after injection are basically the same as that of Example 1. The morphology of the sample of Comparative Example 4 after injection is as shown in panel B of FIG. 3; and the morphology of the sample of Comparative Example 7 after injection is as shown in panel C of FIG. 3.

**Table 2 Appearance test results**

| Example | State | | Morphology of sample after injection |
|---|---|---|---|
| | State of prepared solution after primary physical semi-cross-linking and swelling | State after low-temperature irradiation cross-linking | |
| Example 1 | White dilute solution with suspended particles | Colorless and transparent jelly-like gel | As shown in panel A of FIG. 3 |
| Example 2 | White dilute solution with suspended particles | Colorless and transparent jelly-like gel | |
| Example 3 | White dilute solution with suspended particles | Colorless and transparent jelly-like gel | |
| Example 4 | White dilute solution with suspended particles | Colorless and transparent jelly-like gel | |
| Example 5 | White dilute solution with suspended particles | Colorless and transparent jelly-like gel | |
| Comparative Example 1 | Milky white clear solution | Milky white clear solution | / |
| Comparative Example 2 | White turbid dilute solution | White turbid dilute solution | / |
| Comparative Example 3 | White dilute solution with suspended particles | White dilute solution with suspended particles | / |
| Comparative Example 4 | White sticky pasty gel | Milky white sticky pasty gel | As shown in panel B of FIG. 3 |
| Comparative Example 5 | White dilute solution with suspended particles | White dilute solution with suspended particles | / |
| Comparative Example 6 | White dilute solution with suspended particles | White dilute solution with suspended particles | / |
| Comparative Example 7 | Milky white opaque gel | Milky white opaque gel | As shown in panel C of FIG. 3 |

| | | | |
|---|---|---|---|
| Note: "/" means that the sample is in a dilute solution state and cannot be molded after injection, and it is in a flowing liquid state. | | | |

4. The pushing force was tested on a universal mechanical testing machine. 2 ml of the sample of Example 1 after filling was fitted with a 27G needle, fixed on a mechanical testing machine, and pushed at a constant speed of 10 mm/min until the sample was pushed out with a constant force value, thus completing the experiment. A curve of the pushing force of the sample over time during pushing was recorded, and the maximum force value recorded during pushing was the pushing force. The results are shown in Table 3. FIG. 4 is a test of the pushing force of the recombinant collagen filler for injection in Example 1. From FIG. 4, it can be derived that the pushing force for the recombinant collagen gel material is approximately close to 16 N.
5. Osmotic pressure: 300 µl of a sample was taken and tested according to General Rule (0632 Osmolarity Determination Method - 1 Determination of Osmolarity), Chinese Pharmacopoeia (2020 Edition, Volume IV). The results are shown in Table 3. The osmotic pressures of the recombinant collagen gel materials prepared in Examples 1-5 are in a range of 270-310 mOsm/L, which is close to the ideal state of human physiology and activity.
6. Rotational viscosity: 200 ml of a sample was taken for viscosity detection by a rotational viscometer at 25°C using a No. 5 rotor at a rotating speed of 25 rpm. The results are shown in Table 3. The rotational viscosities of the recombinant collagen gel materials prepared in Examples 1-5 were in the range of 6000-9000 cp.

**Table 3 Performance detection results of materials prepared by examples and comparative examples**

| | Osmotic pressure (mOsm/L) | Pushing force (N) | Rotational viscosity (cp) |
|---|---|---|---|
| Example 1 | 296.0 | 16 | 6500 |
| Example 2 | 298.4 | 17 | 7920 |
| Example 3 | 304.8 | 18 | 8335 |
| Example 4 | 297.3 | 16 | 6755 |
| Example 5 | 299.2 | 16 | 7026 |
| Comparative Example 1 | 279.6 | / | 1006 |
| Comparative Example 2 | 275.5 | / | 1504 |
| Comparative Example 3 | 209.4 | / | 307 |
| Comparative Example 4 | 304.5 | 20N | 9086 |
| Comparative Example 5 | 275.5 | / | 1050 |
| Comparative Example 6 | 270.0 | / | 400 |
| Comparative Example 7 | 308.2 | 25 N | 12360 |

7. Biological evaluation: Test according to GB/T 16886: The samples prepared in Examples 1-5 and Comparative Examples 1-7 were subjected to a cytotoxicity experiment. The results are shown in Table 4. The original leaching solutions of the recombinant collagen gel materials prepared in the comparative examples and examples acted on L929 cells for 24 h, and the cell viabilities were all 85% or more, without obvious cytotoxicity.

**Table 4 Cytotoxicity results**

| | | | | | |
|---|---|---|---|---|---|
| Example | Cell viability | | | | |
| | Blank group | Material | | | |
| Example 1 | | 100% | 50% | 25.00% | 12.50% |
| | 100.00% | 92.45% | 96.78% | 99.99% | 103.21% |
| Example 2 | 100.00% | 95.66% | 100.23% | 104.12% | 108.35% |
| Example 3 | 100.00% | 90.22% | 94.16% | 99.03% | 103.55% |
| Example 4 | 100.00% | 89.66% | 92.67% | 95.56% | 99.21 % |
| Example 5 | 100.00% | 88.16% | 92.11% | 97.42% | 101.40% |
| Comparative Example 1 | 100.00% | 85.10% | 89.22% | 92.56% | 96.98% |
| Comparative Example 2 | 100.00% | 88.28% | 92.86% | 96.48% | 102.00% |
| Comparative Example 3 | 100.00% | 91.02% | 95.33% | 101.00% | 105.43% |
| Comparative Example 4 | 100.00% | 85.28% | 89.55% | 93.55% | 95.04% |
| Comparative Example 5 | 100.00% | 89.22% | 90.34% | 95.22% | 96.21 % |
| Comparative Example 6 | 100.00% | 90.05% | 93.45% | 97.12% | 98.36% |
| Comparative Example 7 | 100.00% | 89.11% | 94.36% | 98.54% | 101.17% |

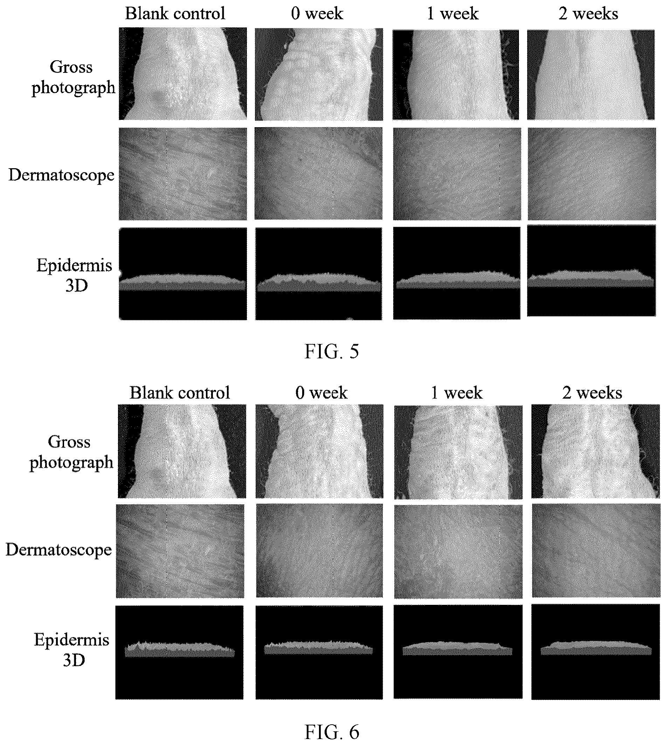

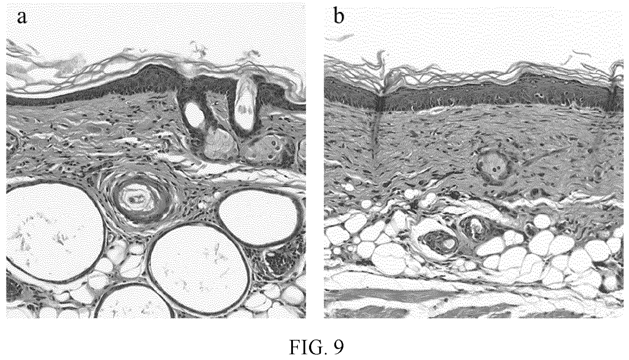

8. Test for therapeutic effect on photoaged model: 1 ml of the samples of Example 1 and Comparative Example 1 after filling were fitted with a 34G needle. The samples were respectively intradermally injected as 1 ml solutions into the dorsal skin of photoaged SKH-1 murine models in a uniform spotted pattern. For the control group, 1 ml of normal saline was intradermally injected into the dorsal skin of photoaged SKH-1 murine models in a uniform spotted pattern. The exposed dorsal skin, the wrinkles of the skin, the pigmentation, and the texture of the dorsal skin of the rats after injection in each group were respectively recorded weekly by a digital camera and a digital microscope at 0 week (0w), 1 week (1w), and 2 weeks (2w). The results are shown in FIGs. 5 and 6. In addition, the skin condition was detected by an assistant skin analyzer (skin analysis system, CBS-806, Wuhan Bosi Electronic Co., Ltd., Wuhan, China). The epidermis was photographed for 3D every week. The skin elasticity, collagen content, and moisture content, as three parameters representing the main parameters of the skin condition, were measured. Three measuring points evenly distributed on the dorsal skin were selected, and each measuring point was measured three times to reduce the measurement error. According to the measured data, the average value was calculated. The results are shown in FIGs. 5 to 8.
9. Histological results of therapeutic effect on photoaged model: At 2w after treatment, the tissues in the injection areas in the blank control group and the experimental group, about 1 cm * 1 cm, were cut off, washed with normal saline, and then laid flat in an embedding box, fixed in 4% formaldehyde for at least 24 hours, then embedded in paraffin, and sliced at 5 µm. The tissue cell and matrix distributions, the presence or absence of inflammation, etc., were observed in the injection areas by HE method (hematoxylin-eosin staining method). The results are shown in FIG. 9, in which panel a in FIG. 9 represents the blank control, and panel b in FIG. 9 represents the experimental group.

### Experimental results

### 1. Results of test for therapeutic effect on photoaged model:

FIGs. 5 and 6 are images of the therapeutic effect of the gel materials prepared in Example 1 and Comparative Example 1 respectively on a photoaged model.

As shown in the results of FIG. 5, compared with the blank control group (normal saline group), the gel material prepared in Example 1 showed reduced wrinkles, a fine skin texture, glow, and relatively flat skin at 2w after injection, indicating that the recombinant collagen gel material prepared in Example 1 can significantly improve the macroscopic performance of skin photoaging.

As shown in the results of FIG. 6, ulceration occurred at the injection site on the skin of murine models at 1w after injection with the gel material prepared in Comparative Example 1. At 2w after injection, compared with the control group (normal saline group), the skin texture was rough and dull, indicating that compared with the material in Example 1, the gel material prepared in Comparative Example 1 had more difficulty in significantly improving the macroscopic performance of skin photoaging.

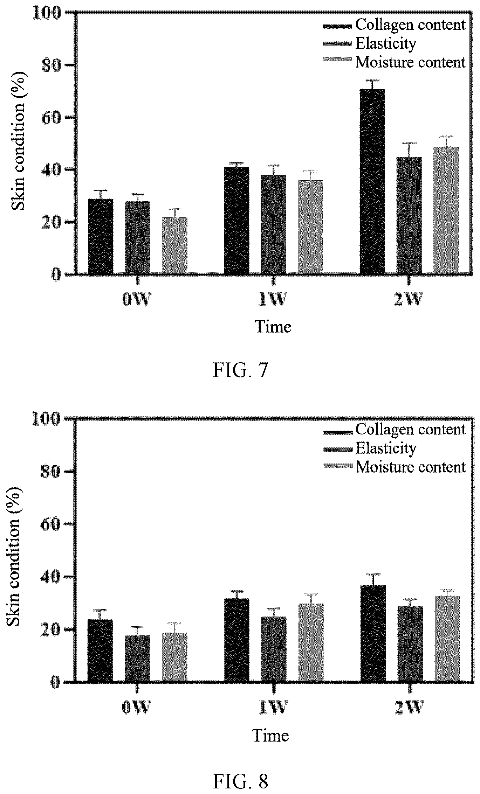

Table. 5 and FIG. 7 are the statistical results of skin elasticity, collagen content, and moisture of a photoaged murine model treated with the recombinant collagen gel material prepared in Example 1 at 0w, 1w, and 2w.

**Table 5 Statistical results of skin elasticity, collagen content, and moisture from Example 1**

| | Collagen content | Skin elasticity | Moisture content |
|---|---|---|---|
| Control group | 25% | 20% | 15% |
| | 20% | 15% | 16% |
| | 22% | 19% | 20% |
| 0w | 27% | 30% | 25% |
| | 33% | 28% | 23% |
| | 28% | 25% | 19% |
| 1w | 39% | 42% | 36% |
| | 41% | 38% | 40% |
| | 42% | 35% | 33% |
| 2w | 70% | 47% | 46% |
| | 74% | 49% | 47% |
| | 68% | 39% | 53% |

As shown in the results of Table 5 and FIG. 7, compared with the control group, the experimental group (the material of Example 1) exhibited a significantly enhanced improvement in collagen content, moisture content, and skin elasticity at 0w, 1w, and 2w, indicating the recombinant collagen gel material prepared in Example 1 can significantly improve the photoaged skin condition of the skin.

Table 6 and FIG. 8 are the statistical results of skin elasticity, collagen content, and moisture of a photoaged murine model treated with the gel material prepared in Comparative Example 1 at 0w, 1w, and 2w.

**Table 6 Statistical results of skin elasticity, collagen content, and moisture from Comparative Example 1**

| | Collagen content | Skin elasticity | Moisture content |
|---|---|---|---|
| Control group | 25% | 17% | 10% |
| | 23% | 19% | 19% |
| | 18% | 14% | 12% |
| 0w | 20% | 20% | 17% |
| | 23% | 18% | 19% |
| | 28% | 15% | 21% |
| 1w | 29% | 22% | 32% |
| | 31% | 27% | 30% |
| | 35% | 26% | 28% |
| 2w | 40% | 30% | 26% |
| | 34% | 27% | 40% |
| | 38% | 29% | 33% |

As shown in the results of Table 6 and FIG. 8, compared with the control group, the experimental group (the material of Comparative Example 1) exhibited no significantly enhanced improvement in collagen content, moisture, and elasticity at 0w, 1w, and 2w, indicating the gel material prepared in Comparative Example 1 cannot significantly improve the photoaged skin condition of the skin.

### 2. Histological results of therapeutic effect on photoaged model:

FIG. 9 shows hematoxylin-eosin staining pictures of an aged murine model treated with the recombinant collagen gel material prepared in Example 1 at 2w. In the figure, panel a in FIG. 9 represents the blank control, and panel b in FIG. 9 represents the experimental group (the material of Example 1). As shown in the histological results of FIG. 9, the recombinant collagen material prepared in Example 1 exhibits good biocompatibility, without obvious inflammation and granuloma after implantation, and the collagen fibers in the experimental group are significantly arranged more tightly than the collagen fibers in the blank control group, indicating that the recombinant collagen gel material prepared by the technical solution of the present disclosure can significantly improve the photoaged skin condition of the skin.

## Claims

1. A preparation method for preparing a recombinant collagen gel material, comprising:
mixing a recombinant collagen with a first aqueous phase to obtain a recombinant collagen solution, and drying and granulating the recombinant collagen solution to obtain a first recombinant collagen microsphere;
physically cross-linking the first recombinant collagen microspheres to obtain a second recombinant collagen microsphere;
mixing, under stirring, the second recombinant collagen microspheres with a second aqueous phase, followed by swelling to obtain a white dilute solution with suspended particles; and
subjecting the white dilute solution with suspended particles to low-temperature irradiation cross-linking to obtain the recombinant collagen gel material.

2. The preparation method according to claim 1, wherein the amino acid sequence of the recombinant collagen is as set forth in SEQ ID No: 1;
preferably, the pH of the recombinant collagen solution is less than 7, more preferably, the pH is 4-6;
preferably, in the recombinant collagen solution, the mass fraction of the recombinant collagen is 10%-20%;
preferably, in the white dilute solution with suspended particles, the mass fraction of the second recombinant collagen microsphere is 2%-4%.

3. The preparation method according to claim 1, wherein the drying and granulating are carried out using a spray dryer with a nozzle diameter of 0.75-1.5 mm at a spray temperature of 140-260°C at a feed rate of 200-500 ml/h.

4. The preparation method according to claim 1, wherein the physical cross-linking is performed at a temperature of 120-160°C for a time period of 2-5 hours.

5. The preparation method according to claim 1, wherein the mixing under stirring is performed at a rotating speed of 80-120 r/min for a time period of 30-60 min;
preferably, the swelling is performed at a temperature of 2-8°C for a time period of 6-10 hours.

6. The preparation method according to claim 1, wherein for the irradiation cross-linking, an electron beam or ⁶⁰Co-γ ray is used, and the irradiation dose is 10-25 kGy;
preferably, during the irradiation cross-linking, the white dilute solution with suspended particles has a temperature of 2-8°C.

7. The preparation method according to claim 1, wherein the first aqueous phase or second aqueous phase is selected from one of, or a combination of two or more of ultrapure water, water for injection, and a phosphate buffer.

8. A recombinant collagen gel material prepared by the preparation method according to any one of claims 1-7.

9. A recombinant collagen gel material, wherein based on a total mass of the recombinant collagen gel material being 100%, the recombinant collagen is comprised at 2%-4%, and the aqueous phase is comprised at 96%-98%;
the recombinant collagen has an amino acid sequence as set forth in SEQ **ID** No: 1; and
the recombinant collagen gel material is a colorless and transparent jelly-like gel with a porous net-like structure inside.

10. The preparation method according to any one of claims 1-7 or the recombinant collagen gel material according to claim 8 or 9 for use in the preparation of a medical material,
preferably, the medical material is used for medical aesthetic water light therapy, medical aesthetic filling, soft tissue repair, or adhesion prevention.
